# EUROPEAN PATENT APPLICATION

(11) **EP 1 640 026 A1**
(43) Date of publication of application: **29.03.2006**
(21) Application number: 04747218.8
(22) Date of filing: 30.06.2004
(51) Int. Cl.: A61L 31/00

(54) **ADHESION INHIBITING MATERIAL FOR VERTEBRAL/SPINAL OPERATION**

(30) Priority: 30.06.2003 JP 2003186760
(71) Applicant: DENKI KAGAKU KOGYO KABUSHIKI KAISHA, Tokyo 103-8338 (JP); Haro, Hirotaka, Tokyo 1138510 (JP)
(72) Inventor: HARO, Hirotaka, Tokyo Medical Dental Uni.Grad.Sch., Tokyo 1138510 (JP); KATO, Takeshi, Tokyo Medical Dental Uni.Grad.Scho., Tokyo 1138510 (JP); MIYOSHI, Teruzou, Denki Kagaku Kogyo Kabushiki K., Machida-shi, Tokyo 1948560 (JP); MIYATA, Yoshiaki, Denki Kagaku Kogyo Kabushiki K., Machida-shi, Tokyo 1948560 (JP); UMEDA, Toshihiko, Denki Kagaku Kogyo Kabushiki K., Machida-shi, Tokyo 1948560 (JP)
(74) Representative: Hartz, Nikolai
(86) International application number: PCT/JP2004/009750
(87) International publication number: WO 2005/000374

(57) **Abstract**

It is to provide a material to inhibit spine/spinal cord adhesion in the form of a sponge, a film or a suspension to be used for the purpose of assisting/accelerating the tissue healing. An adhesion inhibiting material for a spine/spinal cord surgery in the form of a sponge, a film or a suspension to be used for reducing the degree of adhesion or for inhibiting adhesion caused by a spine/spinal cord surgery, which contains a crosslinked acid polysaccharide.

## Description

### TECHNICAL FIELD

The present invention relates to a material to inhibit adhesion of spine/spinal cord in the form of a sponge, a film or a suspension to be used for the purpose of assisting or accelerating tissue healing.

### BACKGROUND ART

Adhesion can be defined as adhesion between tissues which should originally be present distant from each other. The causes may be not only direct surgical injury on the tissue by a surgery but also talc coated on surgical gloves and tissue fragments of e.g. gauze or cotton to be used for treatment. Adhesion is said to be observed in 80% or more of surgical operations, and in general surgery, it may cause motility disorder of an organ, obstipation, etc. Further, in a spine/spinal surgery, it is highly possible that adhesion causes serious symptoms such as cauda equine syndrome, arachnoiditis or radiculopathy by cauda equina adhesion, and myelopathy by dural adhesion, and in a case where a reoperation is required, it is required to remove the adhesion before an originally required treatment is carried out, and such is a burden on a surgeon and may cause a nerve damage by scar removal during the operation as well. In the spine/spinal cord surgery, the surgical operation involves an operation at a portion close to nerves, and accordingly if there is adhesion with e.g. dura mater around the nerves, nerve damage may be caused during the operation. For example, in a decompression operation against disk herniation, damage on the nerve tissue has to be avoided. However, in a case of recurrent hernia, adhesion may occur on the dura mater or the like after the first operation, and operation in the reoperation tends to be difficult, thus causing a nerve damage in some cases. Accordingly, inhibition of adhesion after the surgery in the spine/spinal cord region not only makes the medical treatment effective but also leads to prevention of an artificial damage on the central nerve system and thus prevention of intractable adhesive arachnoiditis.

Adhesion is considered to be formed by six steps ("The Japanese Journal of Artificial Organs", 1994 to 1995, 282 to 285). Namely, the body fluid, the blood or the like flows to a portion abnormalized by the damage by e.g. a surgery or inflammation, and fibrinogen exudes to form fibrin and thereby to form a fibrin network. This process progresses by the minute. Then, by the hour, inflammatory cells such as leukocytes, macrophages and fibroblasts invade the fibrin network, and the fibrin network is dissolved by various enzymes discharged from these cells. Then, the invasion of the fibroblasts will be activated by various growth factors discharged from these cells, and the fibrin network around the cells are replaced with collagen fibers. This process proceeds by the day, but this process is irreversible since the basic structure of the adhesion tissue is formed. Further, by the week, a cell fibrous tissue having a complicated collagen assembly is formed, and due to auxotrophy of these cells, countless blood capillaries invade to form a granular tissue. Then, by the month, a part of the tissue undergoes a vulnerary step, but the most of the remaining tissue becomes a collagen tissue, and becomes in a state called a scar tissue which is a very strong and tough tissue. Then, by the year, the amount of collagen in the scar tissue is reduced to cause "twitch" in the peripheral tissues.

The means of preventing the adhesion is considered to segregate tissues or organs which may undergo adhesion, to suppress processes of fibrin deposition, invasion of cells, collagen tissue production, etc. at stages before the above-described irreversible state, or to frequently move tissues which are positionally likely to undergo adhesion not to maintain the contact state. In general, regeneration of mesothelial cells after the serous membrane is damaged is considered to require five to eight days, and when the damaged tissue and the peripheral tissues are physically segregated to prevent their contact during the above time period, prevention or reduction of formation of the adhesion can be expected. On the other hand, in the case of the spine/spinal cord surgery, it takes at least two weeks for the inflammation after the spinal cord surgery to subside, and segregation during that time period is considered to be necessary.

Since adhesion is tissue recovery which is one of physiological phenomena, and it is required to prevent adhesion only at a portion which should not undergo adhesion. Accordingly, it is desirable to segregate tissues or organs which may undergo adhesion. The segregation method is roughly classified into two methods. That is, a method by a floating effect of keeping an organ containing injury in a nonspecifically floating state and a method by a barrier effect of shielding only the injury and its peripheral portion from other tissues may be mentioned.

As an adhesion inhibiting material by the floating effect of filling the abdominal cavity with a highly viscous product such as a gel or a high concentration solution represented by a polymer material, for example, sodium alginate as disclosed in JP-A-57-167919, sodium chondroitin sulfate as disclosed in Japanese Patent No. 2953702, polymer dextran ("The Medical Frontline" by Tatsuo Tachizaki, Vol. 44, page 645, 1989; "Obstet. Gynecol. (Tokyo) by Hideto Gomibuchi; Oyo Yakuri (Pharmacometrics) by Sachiko Goto et al., Vol. 35, page 359, 1988; JP-A-8-157378), chitosan as disclosed in JP-A-10-502663, and a polymer polysaccharide such as sodium hyaluronate as disclosed in JP-A-8-157378, are found to be effective. However, a drug solution thereof, which has a high viscosity, very slowly moves in a feed tube or a catheter, and accordingly it has to be forcibly injected or it can not sufficiently be injected to the abdominal cavity. Further, it is highly possible that enterobacteria which leak from "suture holes" which are very small holes formed when the intestine or the like is anastomosed by a sewing needle, spread to the abdominal cavity with the highly viscous product as a foothold to cause infection diseases, and such is a serious problem in practical surgery. Application of such an adhesion inhibiting material to the spine/spinal cord surgery can not be said to be practical due to poor operation efficiency, although the problem of the infection diseases tends to be reduced in view of the environment of the periphery of the operation portion.

On the other hand, an adhesion inhibiting material by the barrier effect to be applied to an injured portion and its peripheral portion is less likely to induce infection diseases and is considered to be suitable for general surgery. JP-A-1-301624, U.S. Patent 5,906,997 and the like disclose an adhesion inhibiting material comprising a carboxymethylcellulose composition employing a chemical crosslinking agent or a chemical modifier. Further, a film-form adhesion inhibiting agent "Seprafilm" (manufactured by Genzyme Corporation) is commercially available, which is a composition made by modifying hyaluronic acid and carboxymethylcellulose by a carbodiimide, developed based on JP-A-5-508161 and JP-A-6-508169. On the other hand, as an example of use of carboxymethylcellulose which employs no crosslinking agent nor modifier, i.e. which is not substantially modified, as an adhesion inhibiting material, a film-form Oxiplex (manufactured by FizioMed) is commercially available in the United States, which is made of a carboxymethylcellulose and a polyethylene oxide, developed based on U.S. Patent 5,906,997, U.S. Patent 6,017,301 and U.S. Patent 6,034,140. However, no example showing effectiveness of such an adhesion inhibiting material in the spine/spinal cord surgery has been disclosed.

As a treatment to prevent adhesion at the time of the spine/spinal cord surgery, use of the patient's own subcutaneous fat has been proposed from the late 1970's (Vopr. Neirokhir., Jul-Aug (4), 1976, etc.). Vascular or avascular subcutaneous fat in the vicinity of the operation portion is applied to a defective or injured portion by the surgery. Although it is not necessary to consider the immune response since the self tissue is used, it is required to take the subcutaneous fat, which increases a process to remove the tissue for the surgeon. Further, Clin. Orthop, 215 (1987) reported use of porcine dermis, and Japanese Patent No. 2905718 and Japanese Patent No. 2905719 disclose use of human amnion. In the former, which employs a tissue from a different species, a chemical substance to be used for a treatment against the immune response or to be used to destroy antigenecity of the dermis may remain. The latter possesses an ethical problem, and the human amnion is hardly a practical adhesion inhibiting material.

On the other hand, a silicon rubber as disclosed in German Patent No. 2,722,563, a polyether-polyester copolymer as disclosed in U.S. Patent 5,480,436, VICRYL (Polyglactin 910) mesh as reported in J. Nuerosurgery, 63 (3), 1985, Dacron as reported in Yonsei Med. J., 31 (4), 1990, and a spine/spinal cord adhesion inhibiting material employing a polytetrafluoroethylene as disclosed in JP-A-10-244611, have also been disclosed or reported. However, since such adhesion inhibiting materials have no or poor bioadhesion properties, a further treatment such as suturing may be required to segregate the operated portion and other tissues. This not only increases a burden on a surgeon but also entails a risk of another adhesion to be induced by bleeding or by use of an adhesive at the time of the treatment such as suturing.

For the purpose of improving adhesion properties or residual properties to the application site, U.S. Patent 5,056,839 discloses a crosslinked product of carboxymethylcellulose and a polyethylene oxide by dimethylurea, and JP-A-2001-278984 discloses a gel comprising an esterified carboxyl group-containing polysaccharide and a compound having two or more α-amino groups. These products employing a biocompatible polymer material are expected to have favorable tissue affinity and tissue adhesion properties, but the influence of the remaining chemical substance to be used for formation of a crosslinked product or a gel over the nerves or the like is considered.

On the other hand, as an adhesion inhibiting material to be used in the spine/spinal cord region employing collagen or gelatin, for example, JP-A-2002-10376, U.S. Patent 6,221,109 and WO02/022184 disclose an adhesion inhibiting material employing collagen and U.S. Patent 6,066,325 discloses an adhesion inhibiting material employing gelatin. Further, effectiveness of materials called "ADCON-L" and "Gel Amidon Oxyde (GAO)" is also reported in Eur. Spine J., Vol. 5, 1996, Neurosurgery, Vol. 38, 1996, Am. J. Orthop., Vol. 27, 1998, Neurosurgery, Vol. 50, 2002 and J. Neurosurg., Vol. 97, 2002. Such adhesion inhibiting materials are used as they are or as crosslinked products employing a crosslinking agent, but since the collagen and the gelatin as their raw materials are biological products, they have difficulty in their further stable supply considering the registration trend, etc.

As a gel having biocompatibility and containing substantially no chemical crosslinking agent nor modifier, a polyvinyl alcohol hydrogel as disclosed in European Patent No. 107,055, water insoluble hyaluronic acid as disclosed in WO99/10385 and water insoluble carboxymethylcellulose as disclosed in WO01/34214 are mentioned. However, no example wherein such a gel or water insoluble compound is used as an adhesion inhibiting material in the spine/spinal cord surgery has been disclosed.

The spinal cord which plays a role as a center of nerves is covered with the pia mater, the arachnoid and the dura mater, they are covered with a fat layer and the periosteum, and they are accommodated in the vertebral canal comprising the vertebral body and the vertebral arch, and a certain space is maintained between the pia mater and the vertebral arch. Accordingly, it is important that the adhesion inhibiting material to be employed prevents adhesion by maintaining the above space even after the surgical treatment, and the adhesion inhibiting material is also required to inhibit thickening of the dura mater and the periosteum. Further, when a decompression operation or the like is carried out, inflammation has already appeared at a portion to be operated in many cases, and the adhesion inhibiting material preferably has an anti-inflammatory effect. However, an adhesion inhibiting material having such properties to be used in the spine/spinal cord region has not been found yet.

On the other hand, in view of application of medical devices in surgery, some devices are colored for the purpose of identification of the application site. A surgical suture is a typical example, and for example, coated VICRYL manufactured by Johnson & Johnson and Surgical Silk are colored violet, black or blue, so as to facilitate identification of the sutured portion even in the presence of the blood or the body fluid. JP-A-3-502704 discloses a film obtained by modifying hyaluronic acid and carboxymethylcellulose with a carbodiimide and colored by Brilliant Blue R, but, no commercially available colored adhesion inhibiting material has been found, and an adhesion inhibiting material with which the application site is easily identified has been desired.

### DISCLOSURE OF THE INVENTION

The present inventors have conducted extensive studies on a safe adhesion inhibiting material to be used in the spine/spinal cord region having high biocompatibility. As a result, they have found an adhesion inhibiting effect of a crosslinked acid polysaccharide which has not substantially been modified, in the spine/spinal cord region, and accomplished the present invention. Further, by coloring the crosslinked acid polysaccharide, observation of a state where the product of the present invention is applied to the operation portion becomes easy.

Namely, the present invention provides (1) an adhesion inhibiting material for a spine/spinal cord surgery in the form of a sponge, a film or a suspension to be used for reducing the degree of adhesion or for inhibiting adhesion caused by a spine/spinal cord surgery, which contains a crosslinked acid polysaccharide, (2) the adhesion inhibiting material according to (1), wherein the time until the degree of dissolution of the crosslinked acid polysaccharide becomes 50% in a phosphate buffered physiological saline (pH 7.4) at 60°C, is at least 15 hours, (3) the adhesion inhibiting material according to (2), wherein the acid polysaccharide is hyaluronic acid and/or carboxymethylcellulose, (4) the adhesion inhibiting material according to any one of (1) to (3), wherein the crosslinking structure of the crosslinked acid polysaccharide is an ester bond, (5) the adhesion inhibiting material according to (4), wherein the crosslinking structure of the crosslinked acid polysaccharide is a self-crosslinking ester bond, (6) the adhesion inhibiting material according to any one of (1) to (5), which has a thickness of from 2 mm to 10 mm in the form of a dry sponge, (7) the adhesion inhibiting material according to any one of (1) to (6), which has a pore size of from 50 µm to 200 µm in the form of a dry sponge, (8) the adhesion inhibiting material according to any one of (1) to (5), which has a thickness of from 50 µm to 1 mm in the form of a dry film, (9) the adhesion inhibiting material according to any one of (1) to (5), wherein the crosslinked acid polysaccharide contained in the suspension has an average particle size of from 100 µm to 1 mm, (10) the adhesion inhibiting material according to any one of (1) to (9), which is colored so as to facilitate identification of the application site of the adhesion inhibiting material.

### BRIEF DESCRIPTION OF THE DRAWINGS

Fig. 1 is a photograph of a hematoxylin-eosin-stained control portion two weeks after an operation.
Fig. 2 is a photograph of a hematoxylin-eosin-stained portion to which crosslinked hyaluronic acid in the form of a sponge was applied two weeks after an operation.
Fig. 3 illustrates spaces between the scar tissue and the dura mater at a control portion and at a portion to which crosslinked hyaluronic acid in the form of a sponge was applied after an operation.
Fig. 4 illustrates the numbers of inflammatory cells per unit area with high power of tissue preparations at a control portion and at a portion to which crosslinked hyaluronic acid in the form of a sponge was applied after an operation.
Fig. 5 illustrates thicknesses of the dura mater in tissue preparations at a control portion and at a portion to which crosslinked hyaluronic acid in the form of a sponge was applied after an operation.

### EXPLANATION OF SYMBOLS

1: Remaining vertebral arch, 2: Scar formed after removed of the vertebral arch, 3: Spinal cord, 4: Remaining hyaluronic acid gel

### BEST MODE FOR CARRYING OUT THE INVENTION

Now, the present invention will be explained in detail below.

The adhesion inhibiting material of the present invention means one which is bonded or left at an incised or removed portion at the time of a spine/spinal cord surgery so as to reduce the degree of adhesion or to inhibit adhesion to be caused at the operation site or at a peripheral portion thereof. For example, in the case of a disk hernia operation, it means one applicable not only to an inflammation portion by the hernia but also to a surgically injured portion and a peripheral portion thereof caused on the intervertebral disk or the nerve tissue. Further, it means one having biocompatibility since it is applied to the body.

The acid polysaccharide to be used in the present invention means a polymer compound having no adverse effect such as an inflammatory effect or an injurious effect on the tissue. The acid polysaccharide may be either natural product or synthetic product, and it is not limited so long as it becomes water insoluble by crosslinking and it has biocompatibility.

Typical examples of a natural acid polysaccharide include hyaluronic acid and hyaluronates, glycosaminoglycans except for hyaluronic acid and hyaluronates (such as heparin, heparan sulfate and dermatan sulfate), chondroitin sulfate such as (chondroitin-6-sulfate), keratin sulfate and a polylactic acid. Further, typical examples of a synthetic acid polysaccharide include carboxymethylcellulose and carboxymethylcellulose salts which are used also as a suspending agent or a thickener of pharmaceutical products. However, the present invention is by no means restricted to such polymer compounds.

The adhesion inhibiting material in the form of a sponge in the present invention means an adhesion inhibiting material obtained by drying the obtained crosslinked acid polysaccharide by vacuum freeze drying, and the adhesion inhibiting material in the form of a film means an adhesion inhibiting material obtained by drying the obtained crosslinked acid polysaccharide under normal pressure at a temperature of from room temperature to 50°C. Further, the adhesion inhibiting material in the form of a suspension means an adhesion inhibiting material obtained by pulverizing the obtained crosslinked acid polysaccharide in a pharmaceutically acceptable aqueous solvent by means of a homogenizer or by means of ultrasonic treatment, etc.

The phosphate buffered physiological saline to be used in the present invention is preferably a pharmaceutically acceptable solution considering the application of the crosslinked acid polysaccharide to the body, and specifically, preferred is potassium/sodium phosphate buffered physiological saline (pH 7.4) which is commonly used for cell culture.

In the present invention, the time until the degree of dissolution of the crosslinked acid polysaccharide becomes 50% means a time until, when the crosslinked polysaccharide is left to stand in a phosphate buffered physiological saline (pH 7.4) at 60°C, the amount of the acid polysaccharide dissolved from the crosslinked acid polysaccharide becomes 50% of the crosslinked acid polysaccharide before the dissolution (hereinafter referred to as "solubility half life").

In a case where the solubility half life is short, the adhesion inhibiting material tends to be stay at the application site in a short time, and it tends to be difficult to segregate tissues or organs at stages before the irreversible state among adhesion formation steps, and thus no adhesion inhibiting effect can be expected. On the other hand, if the solubility half life is too long, the irreversible state where inflammatory cells, macrophages and fibroblasts invade the crosslinked acid polysaccharide to form the basic structure of the adhesion tissue, tends to be maintained, whereby formation of adhesion is rather accelerated. The solubility half life can easily be adjusted by selecting reaction conditions to obtain the crosslinked acid polysaccharide, and a crosslinked acid polysaccharide having a desired solubility half life can be obtained by adjusting, for example, the concentration of the acid polysaccharide in a solution, the reaction time, the reaction temperature, etc. Since the adhesion inhibiting material is applied to a portion where the peripheral tissues will not significantly move, the solubility half life to obtain retention which the product of the present invention is required to have, is preferably at least 15 hours, more preferably from 20 hours to 30 hours.

The ester bond as the crosslinking structure of the crosslinked acid polysaccharide of the present invention may, for example, be an ester of a polyhydric alcohol with a carboxyl group, an ester of a polyhydric carboxylic acid with a hydroxyl group or an ester of polyhydric epoxy compound with a carboxyl group.

The self-crosslinking ester bond as the crosslinking structure of the crosslinked acid polysaccharide of the present invention means that an ester bond to be formed is directly formed between molecules which the biocompatible material before crosslinking originally has. As a method for producing a product having self-crosslinking ester bonds, in the case of hyaluronic acid as an example, hyaluronic acid having self-crosslinking ester bonds having some of or all the carboxyl groups esterified by alcohol groups in the same polysaccharide chain or another polysaccharide chain is disclosed in EP0341745B1, hyaluronic acid having self-crosslinking ester bonds formed by carrying out at least one cycle of freezing an aqueous hyaluronic acid solution under acidic conditions and defrosting it, is disclosed in WO99/10385, and hyaluronic acid having self-crosslinking ester bonds formed by mixing hyaluronic acid and an acidic solution and making them coexist at a concentration of at least 5% without freezing, is disclosed in WO01/57093. By the method of freezing an aqueous hyaluronic acid solution under acidic conditions, it is confirmed that although the solubility half life increases along with an increase in the freezing time, an excessive freezing time makes hyaluronic acid have a low molecular weight and shortens the solubility half life on the contrary.

With respect to the thickness of the adhesion inhibiting material in the form of a sponge of the present invention, if the sponge is thin, its strength tends to be weak and thus the adhesion inhibiting material is likely to be broken or bent in an application employing tweezers or the like. Further; if it is thick, although its strength can be kept, it is estimated to be difficult to adjust the size to be fitted with the size of the defective portion, or the adhesion inhibiting material may not sufficiently cover the defective portion or may be applied even to an unnecessary portion due to the volume change by absorption of the body fluid. Accordingly, the thickness of the adhesion inhibiting material in the form of a dry sponge is preferably from 2 mm to 10 mm. Further, it is also possible to overlay thin products of the present invention.

In a case where the sponge of the present invention has a small pore size, the density of the biocompatible material tends to be high and the sponge tends to be hard, and such is unfavorable considering the influence over the nerves. On the other hand, in a case where the pore size is large, a soft sponge will be obtained, but the handling efficiency tends to decrease in the same manner as in the case of the thickness. Accordingly, the pore size of the sponge is preferably from 50 µm to 200 µm as dried.

The thickness of the adhesion inhibiting material in the form of a film of the present invention, is considered in the same manner as in the case of the adhesion inhibiting material in the form of a sponge. Namely, if the film is thin, the handling efficiency tends to decrease since the film strength is low, and if it is thick, the film strength is high, whereby the film is likely to damage the nerves or the like when it is applied. Accordingly, the thickness of the adhesion inhibiting material in the form of a dry film is preferably form 50 µm to 1 mm. Further, it is possible to overlay thin films of the adhesion inhibiting material.

The average particle size of the crosslinked acid polysaccharide contained in the adhesion inhibiting material in the form of a suspension is determined depending upon the operation efficiency at the time of application, the adhesion properties to the tissue, easiness in the production step, etc. In a case where the average particle size is small, favorable operation efficiency at the time of application will be achieved even at a high concentration of the crosslinked acid polysaccharide, but a plurality of pulverizing steps will be required in many cases. On the other hand, in a case where the average particle size is large, preparation is possible only by pulverization by means of a cup mixer or the like, but it is estimated that the operation efficiency at the time of application at a high concentration tends to be poor. Further, the adhesion properties to the tissue are influenced not only by the average particles size but also the type, concentration, etc. of the biocompatible material to be contained, and it is preferred that these factors are easily adjusted. Accordingly, the average particle size is preferably from 100 µm to 1 mm at which the production is easy and favorable operation efficiency at the time of application will be achieved.

The concentration of the crosslinked acid polysaccharide in the suspension is not particularly limited, but if the concentration is low, the application may be inhomogeneous due to separation of a gel portion and a solution portion, and the adhesion properties of the crosslinked acid polysaccharide to the application site may be insufficient. On the other hand, if the concentration is high, it is considered that the capacity of a surgeon is required for application, and the operation efficiency at the time of an operation may be deteriorated. Accordingly, the concentration is suitably determined depending upon the type of the acid polysaccharide to be employed, the average particles size of the crosslinked acid polysaccharide, and the type and the concentration of a salt to be used for the solution.

As the form of application, in view of easiness in the production step, handling efficiency at the time of application and adhesion properties to the tissue, a film is preferred to a suspension, and it is preferred to a sponge.

In the present invention, hyaluronic acid obtained by extraction from animal tissues or by fermentation may be used without any restriction on its source. However, hyaluronic acid obtained by fermentation which is not included in biological products is preferred.

The strain used in fermentation is preferably a hyaluronic acid-producing microorganism isolated from nature such as the genus Streptococcus or a mutant which steadily produces hyaluronic acid in high yield such as Streptococcus equi FM-100 (accession number 9027 given by National Institute of Bioscience and Human-Technology) disclosed in JP-A-63-123392 or Streptococcus equi FM-300 (accession number 2319 given by National Institute of Bioscience and Human-Technology) disclosed in JP-A-2-234689. Pure hyaluronic acid obtained from cultures of the above-mentioned mutants may be used.

The molecular weight (weight average molecular weight, the same applies hereinafter) of the hyaluronic acid to be used in the present invention is preferably within a range of from about 1×10⁵ to about 1×10⁷ Da. Hyaluronic acid having a higher molecular weight may also be used after the molecular weight is lowered into this range by treatment such as hydrolysis. In the present invention, the concept of hyaluronic acid is used so as to include its alkali metal salts such as sodium, potassium lithium and calcium salts, too.

The molecular weight of the carboxymethylcellulose to be used in the present invention is not particularly limited, but is preferably within a range of from about 1×10⁴ to about 5×10⁵ Da. Carboxymethylcellulose having a higher molecular weight may also be used after the molecular weight is lowered into this range by treatment such as hydrolysis.

With respect to the etherification degree which is another parameter of the carboxymethylcellulose, its use is by no means restricted within a range where it becomes insoluble in water. In the present invention, the concept of carboxymethylcellulose is used so as to include its alkali metal salts such as sodium, potassium, lithium and calcium salts, too.

It is possible to obtain a crosslinked acid polysaccharide by e.g. a method of freezing and defrosting an acidic solution of an acid polysaccharide, or a method of mixing an acid polysaccharide with an acid solution at a concentration of at least 5 mass% and leaving the mixture at a non-freezing temperature, as in the above-described example of hyaluronic acid. Among them, the method of freezing and defrosting an acidic solution is simple and makes it possible to obtain a large amount of a crosslinked acid polysaccharide. This method has such advantages that it is possible to control the solubility half life of the crosslinked acid polysaccharide by suitably selecting conditions such as the molecular weight of the acid polysaccharide to be employed, the concentration of the acid polysaccharide in the acidic solution, and the freezing time or the leaving time, and that the crosslinked acid polysaccharide to be obtained is easily be formed into various shapes by selecting the shape of the container at the time of freezing. Further, it is possible to combine products having different shapes.

Now, the sterilization treatment required for medical devices will be described below. Since hyaluronic acid is highly susceptible to radiation, a sponge comprising crosslinked hyaluronic acid can be sterilized by means of an autoclave. Further, it is possible to sterilize a hyaluronic acid solution at a high temperature in a short time and to carry out an acidification step and the subsequent steps under aseptic conditions. On the other hand, since the sugar chain structure of carboxymethylcellulose is relatively stable against heat, radiation, etc., various sterilization methods such as Υ ray sterilization, electron beam sterilization, ethylene oxide gas sterilization, plasma gas sterilization or autoclave sterilization may be applied to a sponge comprising crosslinked carboxymethylcellulose. A change of a solubility in vitro and thus a change of in vivo retention of the crosslinked hyaluronic acid or the crosslinked carboxymethylcellulose caused by a severe treatment of such sterilization are confirmed. However, it is possible to preliminarily produce crosslinked hyaluronic acid or crosslinked carboxymethylcellulose which is more stable i.e. which has a longer solution half life by changing the production conditions thereby to control the in vivo retention after the sterilization treatment.

In a case where the product of the present invention is colored, it is necessary to employ a dye the safety of which when parenterally administered is confirmed. For example, coloring by licorice extract, iron sesquioxide, sodium copper chlorophyllin, permanent violet R special, Bengala and medicinal charcoal as disclosed in Japanese Pharmaceutical Excipients Dictionary (published by Japan Phermaceutical Excipients Counsil), or so-called "Violet 2015" which is practically used for coloring surgical suture is possible. The color is optionally determined depending upon the application site and the application method and is by no means restricted.

Now, the present invention will explained in further detail with reference to Examples. However, the present invention is no means restricted to these specific Examples.

### EXAMPLE 1

Sodium hyaluronate having a molecular weight of 2×10⁶ Da was dissolved in distilled water to prepare a 2 mass% hyaluronic acid aqueous solution. The pH of this aqueous solution was adjusted to pH 1.5 with 1 mol/liter hydrochloric acid. The acidic aqueous solution of hyaluronic acid was put in a 9 cm × 9 cm × 1 cm square polystyrene petri dish, left to stand in a freezer set at -20°C for 10 days and then defrosted at 25°C. Then, washing with distilled water and washing with a phosphate buffer (pH 6.8) at a concentration of 100 mmol/liter were carried out, and drying by freeze drying was carried out. As a result, crosslinked hyaluronic acid in the form of a sponge having a thickness of about 4 mm and a pore size of 120 ± 45 µm as dried was obtained.

### EXAMPLE 2

Crosslinked hyaluronic acid in the form of a sponge having a thickness of about 4 mm and a pore size of 100 ± 40 µm as dried was obtained in the same manner as in Example 1 except that the acidic aqueous solution of hyaluronic acid was left to stand in a freezer set at -20°C for 20 days.

### EXAMPLE 3

Crosslinked hyaluronic acid in the form of a sponge having a thickness of about 7 mm and a pore size of 135 ± 45 µm as dried was obtained in the same manner as in Example 1 except that the acidic aqueous solution of hyaluronic acid was left to stand in a freezer set at -20°C for 30 days.

### EXAMPLE 4

Sodium carboxymethylcellulose having a 1% viscosity of from 1,000 to 2,800 mPa·s at 25°C (etherification degree: 0.65 to 0.95, calculated molecular weight: about 3.0×10⁵ Da, manufactured by Hercules Incorporated) was dissolved in distilled water to prepare a 2 mass% carboxymethylcellulose aqueous solution. The pH of this aqueous solution was adjusted to pH 1.5 with 1 mol/liter hydrochloric acid.

The acidic aqueous solution of carboxymethylcellulose was put in a 9 cm x 9 cm square polystyrene petri dish, left to stand in a freezer set at -20°C for 1 day and then defrosted. Then, washing with distilled water and washing with a phosphate buffer (pH 6.8) at a concentration of 100 mmol/liter were carried out, and drying by freeze drying was carried out. As a result, crosslinked carboxymethylcellulose in the form of a sponge having a thickness of about 4 mm and a pore size of 125 ± 45 µm as dried was obtained.

### EXAMPLE 5

Crosslinked carboxymethylcellulose in the form of a sponge having a thickness of about 3 mm and a pore size of 120 ± 35 µm as dried was obtained in the same manner as in Example 4 except that the acidic aqueous solution of carboxymethylcellulose was left to stand in a freezer set at -20°C for 3 days.

### EXAMPLE 6

### TEST ON SOLUBILITY OF CROSSLINKED ACID POLYSACCHARIDS

A phosphate buffered physiological saline having a pH of 7.4 and having the following composition was prepared.

Phosphate buffered physiological saline
Potassium chloride 0.02 mass%
Potassium dihydrogen phosphate 0.02 mass%
Disodium phosphate dodecahydrate 0.29 mass%
Sodium chloride 0.81 mass%

Each of the crosslinked hyaluronic acids and the crosslinked carboxymethylcelluloses obtained in Examples 1, 2, 3, 4 and 5 was immersed in 50 ml of the phosphate buffered physiological saline based on 50 mg of dry hyaluronic acid or carboxymethylcellulose contained in the crosslinked hyaluronic acid or the crosslinked carboxymethylcellulose. The degree of the dissolution of hyaluronic acid or carboxymethylcellulose in the phosphate buffered physiological saline at 60°C was obtained from the concentration of hyaluronic acid or the concentration of carboxymethylcellulose in the phosphate buffered physiological saline.

Namely, the solubility of a crosslinked acid polysaccharide in a neutral aqueous solution at 60°C is defined according to the above test.

### MEASUREMENT OF ACID POLYSACCHARIDE CONCENTRATION

The concentration of hyaluronic acid or carboxymethylcellulose in the phosphate buffered physiological saline was obtained from the peak area by means of gel permeation chromatography (GPC) using a differential refractometer as a detector. Namely, samples of the phosphate buffered physiological saline collected at intervals were filtrated through a filter having a pore size of 0.45 µm and then subjected to GPC, and the concentration was calculated by comparing the obtained peak area with the peak area of hyaluronic acid or carboxymethylcellulose the concentration of which has been known.

The results are shown in Table 1.

**TABLE 1**

| Test No. | Degree of dissolution of acid polysaccharide (%) | | | | Remarks |
|---|---|---|---|---|---|
| | After 3 hours | After 5 hours | After 10 hours | After 15 hours | |
| 1 | 0 | 3 | 20 | 48 | Ex. 1 |
| 2 | 0 | 0 | 5 | 9 | Ex. 2 |
| 3 | 5 | 20 | 49 | 71 | Ex. 3 |
| 4 | 22 | 32 | 55 | 68 | Ex. 4 |
| 5 | 4 | 9 | 17 | 44 | Ex. 5 |

For example, in the case of the crosslinked hyaluronic acid obtained in Example 1 in test No. 1, hyaluronic acid was not dissolved after 3 hours, and the degree of dissolution was 3 mass% after 5 hours, 20 mass% after 10 hours and 48 mass% after 15 hours. Namely, 97 mass% of hyaluronic acid remained after 5 hours, and 52 mass% of hyaluronic acid remained after 15 hours, as the crosslinked hyaluronic acid. Similar results were obtained with respect to the crosslinked hyaluronic acids and the crosslinked carboxymethylcelluloses obtained in the other Examples, and it was found that the solubility can be controlled by freezing time. Further, it was found from these degrees of dissolution that the solubility half lives of the crosslinked hyaluronic acids and the crosslinked carboxymethylcelluloses obtained in Examples 1 to 5 were about 15 hours, about 28 hours, about 10 hours, about 8 hours and about 18 hours, respectively.

### EXAMPLE 7

### TEST FOR INHIBITION OF ADHESION BY CROSSLINKED HYALURONIC ACID IN THE FORM OF A SPONGE IN RABBITS

Japanese White rabbits weighing from 2.0 to 2.5 kg were employed. The thoracolumbar vertebra was exposed by back median section, and the vertebral arch was removed in a size of 10 mm × 5 mm from two bodies of vertebra. The dura mater was exposed at one removed portion (control portion), and the crosslinked hyaluronic acid in the form of a sponge obtained in Example 1, 2 or 3 was applied to the other removed portion. The rabbits were killed after two weeks, one month, two months or 6 months, and the tissue sections were prepared from portions where the vertebral arch was removed, and sections were hematoxylin-eosin-stained to observe the state of adhesion to the periphery, etc.

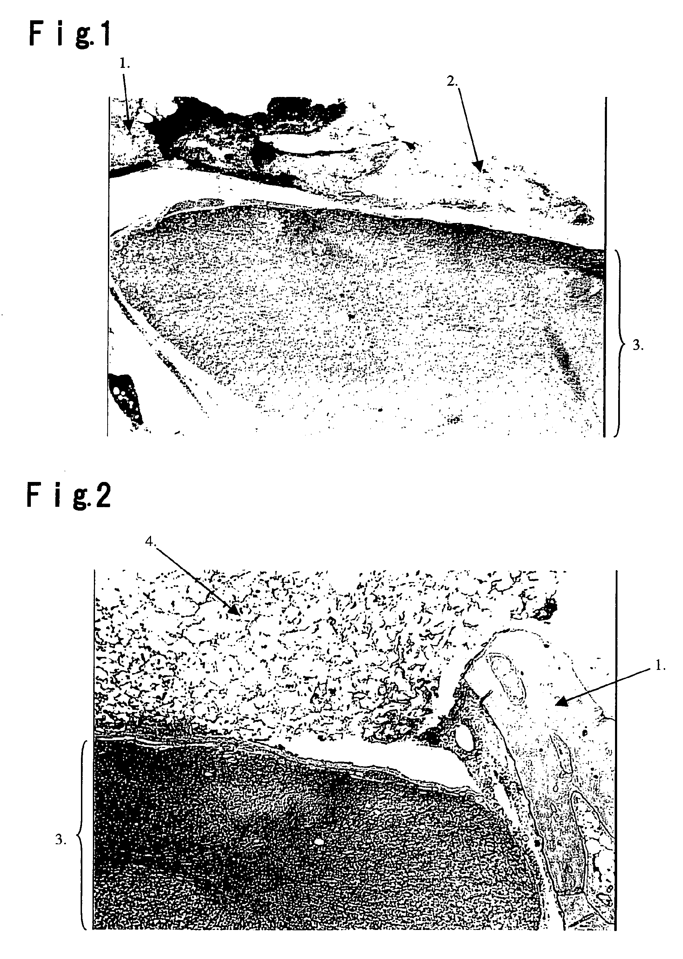

It was observed on the control portion two weeks after the operation that the scar tissue accompanying infiltration of e.g. inflammatory cells and fibroblasts invaded the removed portion, and adhesion to the dura mater was confirmed (Fig. 1). On the contrary, at the portion to which the crosslinked hyaluronic acid in the form of a sponge obtained in Example 1 or 2 was applied, the crosslinked hyaluronic acid was confirmed to remain (Fig. 2) and the infiltration of inflammatory cells into the removed portion significantly decreased as compared with the control portion.

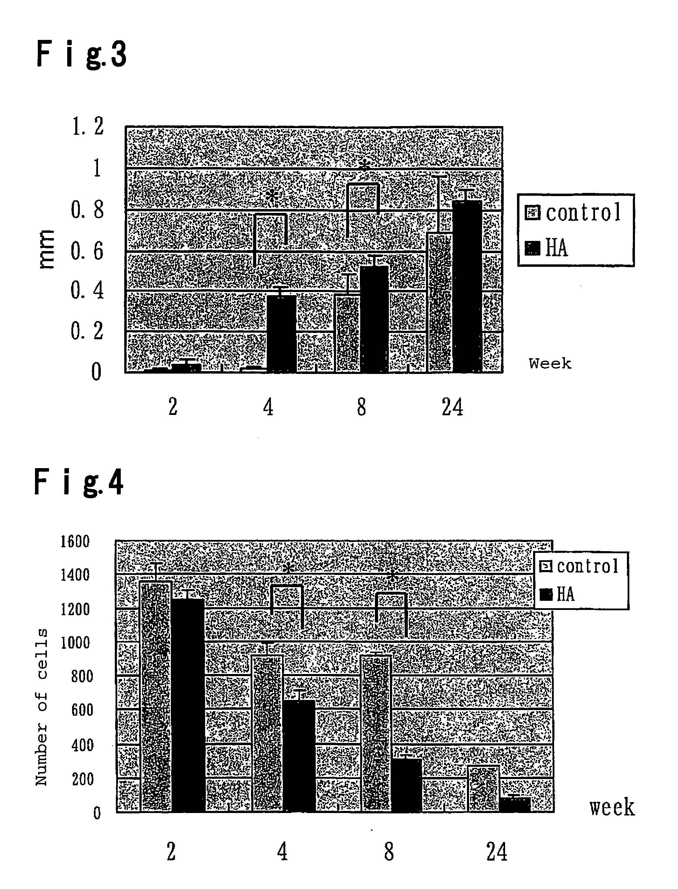

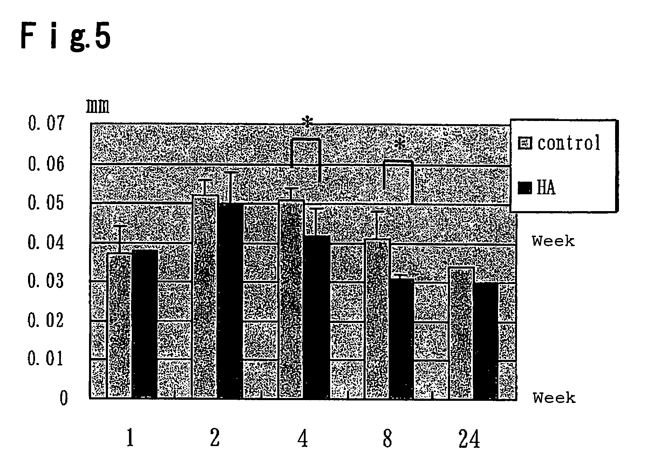

Two months after the operation, the vertebral arch was confirmed to be repaired at each portion, and at a control portions and the portion to which the crosslinked hyaluronic acid in the form of a sponge obtained in Example 3 was applied, adhesion between the dura mater and the repair tissue was confirmed, whereas at portions to which the crosslinked hyaluronic acid in the form of a sponge obtained in Example 1 or 2 was applied, the adhesion was prevented. Remaining of the crosslinked hyaluronic acid was not confirmed at the portions where the adhesion inhibiting effect was confirmed. With respect to the portion to which the crosslinked hyaluronic acid in the form of a sponge obtained in Example 2 was applied, the space between the scar tissue and the dura mater at the portion to which the crosslinked hyaluronic acid in the form of a sponge was applied was significantly larger than that of the control at fourth and eighth weeks after the operation (Fig. 3). Further, with respect to the number of inflammatory cells per unit area with high power in the tissue preparation, the number of invading cells at the portion to which the crosslinked hyaluronic acid in the form of a sponge was applied significantly decreased than that of the control at fourth and eighth weeks after the operation (Fig. 4). Further, the thickness of the dura mater in the tissue preparation where the crosslinked hyaluronic acid in the form of a sponge was applied was significantly thinner than that of the control at fourth and eighth weeks after the operation (Fig. 5). Accordingly, it was found that by application of the crosslinked hyaluronic acid in the form of a sponge, it becomes possible to keep a space between the scar tissue and the dura mater, and it is possible to suppress infiltration of inflammatory cells and thickening of the dura mater.

### EXAMPLE 8

### TEST ON INHIBITION OF ADHESION BY CROSSLINKED CARBOXYMETHYLCELLULOSE IN THE FORM OF A SPONGE IN RABBITS

The same.operation as in Example 7 is carried out except that the crosslinked carboxymethylcellulose in the form of a sponge obtained in Example 4 or 5 is applied to the removed portion in the vertebral arch, and the rabbits are killed after two months and observation is carried out similarly.

At a portion to which the crosslinked carboxymethylcellulose in the form of a sponge obtained in Example 4 is applied, adhesion between the dura mater and the repair tissue is confirmed, and the degree is equal to that at the control portion in Example 7. On the other hand, at a portion to which the crosslinked carboxymethylcellulose in the form of a sponge obtained in Example 5 is applied, no adhesion is confirmed in the same manner as the case where the crosslinked hyaluronic acid in the form of a sponge obtained in Example 1 or 2 was applied.

### EXAMPLE 9

### CYTOTOXITY TEST

A cytotoxity test is carried out with respect to the crosslinked hyaluronic acids in the form of a sponge and the crosslinked carboxymethylcellulose in the form of a sponge obtained in Examples 1, 2 and 5. Namely, the incubation of CCL 1 (NCTC clone 929) cells is carried out in the presence of the crosslinked hyaluronic acid or the crosslinked carboxymethylcellulose obtained in the present invention, and the cytotoxity is evaluated by observing the cell growth behavior. The crosslinked hyaluronic acid in the form of a sponge or the crosslinked carboxymethylcellulose in the form of a sponge obtained in the present invention is soaked in a culture medium for cytotoxity test, and the culture medium is mechanically pulverized, and 20 mg of the pulverized product is loaded on a cell culture insert (pore size: 3 µm) manufactured by Falcon and immersed in the cell culture. For a control experiment, incubation is carried out only with the culture medium for cytotoxity test.
Incubation conditions
Plate: 12-well plate for cell culture
Medium: DMEM medium + 10% fetal bovine serum, 2 ml/well
Temperature: 37°C (under 5% CO₂)
Cell number: 1×10² cells/well

2, 5 and 8 days after incubation, the cell culture is examined on the cell density under an inverted microscope. As a result, it is found that the cell culture grows even in the presence of the crosslinked hyaluronic acid or the crosslinked carboxymethylcellulose as satisfactory as that in the control experiment, and thereby it is ascertained that the crosslinked hyaluronic acid and the crosslinked carboxymethylcellulose obtained in the present invention have no cytotoxity.

### EXAMPLE 10

The same operation as in Example 1 was carried out except that the acidic aqueous solution of hyaluronic acid was left to stand in a freezer set at -20°C for 20 days and then defrosted at 25°C. Then, washing with distilled water and washing with a phosphate buffer (pH 6.8) at a concentration of 100 mmol/liter were carried out, and drying was carried out at 40°C under normal pressure. As a result, crosslinked hyaluronic acid in the form of a film having a thickness of about 500 µm as dried was obtained. In this Example, the solubility half life was about 26 hours.

### EXAMPLE 11

The same operation as in Example 4 was carried out except that the acidic aqueous solution of carboxymethylcellulose was left to stand in a freezer set at -20°C for 3 days and then defrosted at 25°C. Then, washing with distilled water and washing with a phosphate buffer (pH 6.8) at a concentration of 100 mmol/liter were carried out, and drying at about 40°C under normal pressure was carried out. As a result, crosslinked carboxymethylcellulose in the form of a film having a thickness of about 200 µm as dried was obtained.

In this Example, the solubility half life was about 21 hours.

### EXAMPLE 12

### TEST ON EVALUATION OF ADHESION INHIBITING MATERIAL IN THE FORM OF A FILM IN RABBITS

The same operation as in Example 7 is carried out except that the crosslinked hyaluronic acid in the form of a film obtained in Example 10 or the crosslinked carboxymethylcellulose in the form of a film obtained in Example 11 is applied to the removed portion of the vertebral arch, and the rabbits are killed after two months, and observation is carried out similarly.

At a portion to which the crosslinked hyaluronic acid in the form of a film obtained in Example 10 or the crosslinked carboxymethylcellulose in the form of a film obtained in Example 11 is applied, no adhesion is confirmed in the same manner as in the case where the crosslinked hyaluronic acid in the form of a sponge obtained in Example 1 or 2 the crosslinked carboxymethylcellulose in the form of a sponge obtained in Example 5 was applied. Accordingly, it is evident that an adhesion inhibiting effect can be obtained even by the crosslinked hyaluronic acid or the crosslinked carboxymethylcellulose in the form of a film.

### EXAMPLE 13

### EFFECT OF COLORED SPONGE

The crosslinked hyaluronic acid in the form of a sponge obtained in Example 2 was immersed in an aqueous solution containing Alcian blue in an amount of one twentieth by mass ratio, and vacuum dried to obtain a colored sponge. Using this colored sponge, the test on inhibition of adhesion in rabbits as disclosed in Example 7 was carried out, and observation was carried out after two months.

When the sponge was applied, it was easy to identify the crosslinked hyaluronic acid in the form of a sponge even in the presence of the blood thanks to coloring. Further, no adhesion between the dura mater and the repair tissue was confirmed, and no influence by coloring was observed.

### EXAMPLE 14

The crosslinked hyaluronic acid in the form of a sponge obtained in Example 2 was put in a stainless steel cup, and a phosphate buffered physiological saline (pH 6.8) at a concentration of 50 mmol/liter was added so that the hyaluronic acid concentration would be about 1 mass%, followed by pulverization by means of a homogenizer (Nissei Excel Auto Homogenizer DX-11) at 10,000 revolutions/min. The obtained suspension was washed with a physiological saline by means of centrifugal separation three times, to obtain crosslinked hyaluronic acid in the form of a suspension containing about 4.5 mass% of hyaluronic acid in the physiological saline.

### EXAMPLE 15

The crosslinked carboxymethylcellulose in the form of a sponge obtained in Example 5 was put in a stainless steel cup, and a phosphate buffered physiological saline (pH 6.8) at a concentration of 50 mmol/liter was added so that the carboxymethylcellulose concentration would be about 1 mass%, followed by pulverization by means of a homogenizer (Nissei Excel Auto Homogenizer DX-11) at 10,000 revolutions/min. The obtained suspension was washed with a physiological saline by means of centrifugal separation three times, to obtain crosslinked carboxymethylcellulose in the form of a suspension containing about 3.5 mass% of carboxymethylcellulose in the physiological saline.

### EXAMPLE 16

### SOLUBILITY TEST FOR CROSSLINKED ACID POLYSACCHARIDES IN SUSPENSIONS

With respect to the crosslinked hyaluronic acid and the crosslinked carboxymethylcellulose in the suspensions obtained in Examples 14 and 15, the solubility test as disclosed in Example 6 was carried out. As a result, the solubility half life of the crosslinked hyaluronic acid was about 20 hours and the solubility half life of the crosslinked carboxymethylcellulose was about 17 hours, and it was found that the solubility half lives of the crosslinked acid polysaccharides do not substantially change even when a pulverization treatment was carried out.

### EXAMPLE 17

### PARTICLE SIZE OF CROSSLINKED ACID POLYSACCHARIDES IN SUSPENSIONS

With respect to the suspensions obtained in Examples 14 and 15, the particle sizes of the crosslinked hyaluronic acid and the crosslinked carboxymethylcellulose in the suspensions were measured by means of a particle size measuring apparatus (BECKMAN COULTER, model LS 13 320). The crosslinked hyaluronic acid had an average particle size of 409 ± 284 µm, and the crosslinked carboxymethylcellulose had an average particle size of 548 ± 388 µm.

### EXAMPLE 18

### TEST ON INHIBITION OF ADHESION BY CROSSLINKED ACID POLYSACCHARIDES IN THE FORM OF A SUSPENSION IN RABBITS

The same operation as in Example 7 is carried out except that 0.5 ml of the crosslinked hyaluronic acid in the form of a suspension obtained in Example 13 or the crosslinked carboxymethylcellulose in the form of a suspension obtained in Example 14 was applied to the removed portion of the vertebral arch, and the rabbits are killed after one month or after two months, and observation is carried out similarly.

The space between the scar tissue and the dura mater at the application site after one month is 0.2 ± 0.1 in the case of a group to which the crosslinked hyaluronic acid is applied and is 0.3 ± 0.1 in the case of a group to which the crosslinked carboxymethylcellulose is applied, and the distance after two months is 0.4 ± 0.1 in the case of the group to which the crosslinked hyaluronic acid is applied and 0.4 ± 0.1 in the case of the group to which the crosslinked carboxymethylcellulose is applied. Further, no formation of adhesion is confirmed at the application site after two months. Accordingly, it is confirmed that the crosslinked hyaluronic acid in the form of a suspension and the crosslinked carboxymethylcellulose in the form of a suspension have an adhesion inhibiting effect in the same manner as in the case of a sponge or a film.

### EXAMPLE 18

### TEST ON INHIBITION OF ADHESION BY CROSSLINKED HYALURONIC ACID IN THE FORM OF A SPONGE USING RABBIT INFLAMMATORY MODEL

Japanese White rabbits weighing from 2.0 to 2.5 kg were employed. The thoracolumbar vertebra was exposed by means of back median section, and the vertebral arch was removed in a size of 10 mm x 5 mm from two bodies of vertebra. To the portions from which the vertebral arch was removed, human recombinant TNF-α (tumor necrosis factor-α) was applied in an amount of 10 ng/0.5 ml to cause inflammation. The dura mater was exposed at one removed portion (control portion), and the crosslinked hyaluronic acid in the form of a sponge obtained in Example 2 was applied to the other removed portion. The rabbits were killed after two weeks, one month or two months, and tissue sections were prepared from the portions from which the vertebral arch was removed, and the sections were hematoxylin-eosin-stained, to confirm the state of adhesion to the periphery, etc.

The distance between the scar tissue and the dura mater was 0.15 ± 0.15 mm two weeks after the application, and it was 0.4 ± 0.1 mm (control portion: 0.02 ± 0.01 mm) one month after the application and 0.5 ± 0.15 mm (control portion: 0.3 ± 0.02 mm) two months after the application, and a significant difference as compared with the control portion was confirmed. The number of inflammatory cells per unit area with high power (magnification of 40) in the tissue preparation, was 1,200 cells (control portion: 2,000 cells) two weeks after the application, 800 cells (control portion: 1,800 cells) after one month and 500 cells (control portion: 900 cells) after two months, and a significant inflammation inhibiting effect was confirmed even two weeks after the application. Further, the thickness of the dura mater was 60 µm (control portion: 80 µm) two weeks after the application, 45 µm (control portion: 75 µm) after one month and 40 µm (control portion: 55 µm) after two months, and a significant thickening-inhibiting effect was confirmed even two weeks after the application.

As mentioned above, it was found that the product of the present invention has an adhesion inhibiting effect even when applied to an inflammatory site.

### INDUSTRIAL APPLICABILITY

According to the present invention, a crosslinked acid polysaccharide, particularly crosslinked hyaluronic acid and/or crosslinked carboxymethylcellulose in the form of a sponge, a film or a suspension, obtained by employing no chemical crosslinking agent nor chemical modifier, provides an adhesion inhibiting effect in the spine/spinal cord region by controlling its solubility in vitro. Bad influences over biocompatibility resulting from use of a chemical crosslinking agent or a chemical modifier can be avoided, and further, the crosslinked acid polysaccharide is easily cut into a shape suitable for the application site, whereby the crosslinked acid polysaccharide is useful as an adhesion inhibiting material for a surgery.

Further, coloring facilitates identification of the application site, and such is effective to reduce a burden on a surgeon at a final stage of a spine/spinal cord surgery.

## Claims

1. An adhesion inhibiting material for a spine/spinal cord surgery in the form of a sponge, a film or a suspension to be used for reducing the degree of adhesion or for inhibiting adhesion caused by a spine/spinal cord surgery, which contains a crosslinked acid polysaccharide.

2. The adhesion inhibiting material according to Claim 1, wherein the time until the dissolution rate of the crosslinked acid polysaccharide becomes 50% in a phosphate buffered physiological saline (pH 7.4) at 60°C, is at least 15 hours.

3. The adhesion inhibiting material according to Claim 2, wherein the acid polysaccharide is hyaluronic acid and/or carboxymethylcellulose.

4. The adhesion inhibiting material according to any one of Claims 1 to 3, wherein the crosslinking structure of the crosslinked acid polysaccharide is an ester bond.

5. The adhesion inhibiting material according to Claim 4, wherein the crosslinking structure of the crosslinked acid polysaccharide is a self-crosslinking ester bond.

6. The adhesion inhibiting material according to any one of Claims 1 to 5, which has a thickness of from 2 mm to 10 mm in the form of a dry sponge.

7. The adhesion inhibiting material according to any one of Claims 1 to 6, which has a pore size of from 50 µm to 200 µm in the form of a dry sponge.

8. The adhesion inhibiting material according to any one of Claims 1 to 5, which has a thickness of from 50 µm to 1 mm in the form of a dry film.

9. The adhesion inhibiting material according to any one of Claims 1 to 5, wherein the crosslinked acid polysaccharide contained in the suspension has an average particle size of from 100 µm to 1 mm.

10. The adhesion inhibiting material according to any one of Claims 1 to 9, which is colored so as to facilitate identification of the application site of the adhesion inhibiting material.
